# EUROPÄISCHE PATENTSCHRIFT

(11) **EP 0 573 884 B1**
(45) Veröffentlichungstag und Bekanntmachung des Hinweises auf die Patenterteilung: **06.05.1999**
(21) Anmeldenummer: 93108833.0
(22) Anmeldetag: 02.06.1993
(51) Int. Cl.: A61L 29/00, A61M 5/14

(54) **Strahlensterilisierbare recyclierbare Infusions- und Transfusionsgeräte**
Radiation sterilizable recyclable infusion and transfusion devices
Appareil de perfusion ou de transfusion stérilisable par irradiation et recyclable

(30) Priorität: 11.06.1992 DE 4219071
(43) Veröffentlichungstag der Anmeldung: 15.12.1993
(73) Patentinhaber: B. Braun Melsungen AG, 34209 Melsungen (DE)
(72) Erfinder: Herold, Manfred, W-3508 Melsungen (DE); Brethauer, Ullrich, W-3501 Körle (DE)
(74) Vertreter: von Kreisler, Alek, Dipl.-Chem.

(56) Entgegenhaltungen:
- EP-A- 0 355 711
- EP-A- 0 392 214
- EP-A- 0 396 879
- WO-A-92/18173

## Beschreibung

Gegenstand der vorliegenden Erfindung sind strahlensterilisierbare recyclierbare Infusions- und Transfusionsgeräte, deren Bestandteile hergestellt sind aus ausgewählten Polymeren.

Infusions- und Transfusionsgeräte bestehen aus verschiedensten Einzelteilen, die bisher im Stand der Technik aus den verschiedensten Materialien hergestellt worden sind. Die Geräte werden als Einmalartikel hergestellt, da eine Reinigung und erneute Sterilisation praktisch nicht möglich ist, ohne die Eigenschaften der verarbeiteten Kunststoffe mehr oder weniger zu ändern. Bedingt durch die verschiedensten Ausgangsmaterialien war eine Wiederverwendung der einzelnen Kunststoffe bisher nicht möglich.

Einen Überblick über die im Stand der Technik verwendeten Kunststoffe ist in der EP 0 007 601 C2 offenbart. Hier sind Materialien wie Polyvinylchlorid, Polyamid, Polystyrol, Polypropylen, Styrol-Butadien-Styrol, Styrol-Isopren-Styrol, Styrol-Acrylnitril, Acrylnitril-Butadien-Styrol, Styrol-Butadien, Methylmethacrylat-Butadien-Styrol, Methylacrylat-Styrol-Acrylnitril, Polyethylen-Ethylen-Vinylacetat, Polybuten, Polymethylpenten sowie Polyacrylsäureester als Materialien für einzelne Bestandteile von Infusions- und Transfusionsgeräten beschrieben.

In der DE 22 16 987 A1 sind darüberhinaus noch Polyolefine, Polycarbonate, Celluloseester neben Polyvinylchlorid erwähnt.

In der DE 39 14 998 A1 (EP 0 396 879 A2) wird erstmals das Konzept einer einheitlichen Materialzusammensetzung für eine Mehrzahl der Bestandteile der Infusions- und Transfusionsgeräte erwähnt. Unter Verzicht auf PVC Materialien sind sämtliche Bestandteile aus einem Polymeren, Copolymeren oder Blockpolymeren hergestellt. Die jeweils gewünschten Eigenschaften der einzelnen Bestandteile werden durch Modifizierung des Molgewichtes, der Mengenverhältnisse oder der Taktizität gesteuert. Insbesondere wird ein Polystyrol-Homopolymerisat oder als Co- und/oder Blockpolymer ausschließlich Styrol-Butadien-Polymerisat verwendet.

Bisher wurden entsprechende Infusions- und Transfusionsgeräte in der Regel durch die Anwendung von Ethylenoxidgas sterilisiert. Bei der in neuerer Zeit aufkommenden Strahlensterilisierung mit energiereicher Gammastrahlung treten jedoch bei der Mehrzahl der Materialien des Standes der Technik häufig Probleme auf. Insbesondere ist die Eintrübung klar durchsichtiger Bestandteile zu beobachten. Eine hohe Transparenz des gesamten Infusions- und Transfusionsgerätes ist jedoch eine notwendige Voraussetzung und ein wichtiges Kriterium zur Kontrolle der Tropfkammerfüllung, der Einstellgeschwindigkeit des Flüssigkeitsstromes in Abhängigkeit von der Zeit. Bedingt durch die hohe Strahlendosis verringert sich die mechanische Belastbarkeit vieler Materialien, da teilweise sogar Kettenabbaureaktionen induziert werden. Auch treten Geruchsbeeinträchtigungen durch Abbau von Bestandteilen wie Weichmacher oder Vernetzungs-/Vulkanisiermitteln auf.

Darüberhinaus stellt die Flexibilität und das Rückstellvermögen der Schläuche ein besonderes Problem der Strahlensterilisierung dar. Die Schläuche haben normalerweise einem umfangreichen Anforderungsprofil zu entsprechen, denen die bekannten PVC-freien Materialien nicht gewachsen sind.

Außerordentliche Schwierigkeiten bestehen im Stand der Technik zur Gewährleistung einer sicheren Fügung des Tropfkammer-Ober/Unterteiles bzw. aller Schlauchverbindungen mit der Tropfkammer und den Konnektoren zur Erzielung einer flüssigkeits und luftdichten Verbindungsstelle.

Die Aufgabe der vorliegenden Erfindung bestand darin, strahlensterilisierbare recyclierbare Infusions- und Transfusionsgeräte zur Verfügung zu stellen. Weitere Anforderungen an die Infusions- und Transfusionsgeräte sind, daß sämtliche Bestandteile der Geräte chemisch inert sind und die Sterilisierbarkeit mittels Ethylenoxid oder Gammastrahlen ohne Beeinträchtigung der Gebrauchstauglichkeit überstehen. Darüberhinaus ist die vollständige Recyclierbarkeit ohne Zerlegung in die Einzelteile erforderlich. Weiterhin sollen die Infusions- und Transfusionsgeräte ohne Schadstoffentwicklung entsorgbar durch Verbrennung sein.

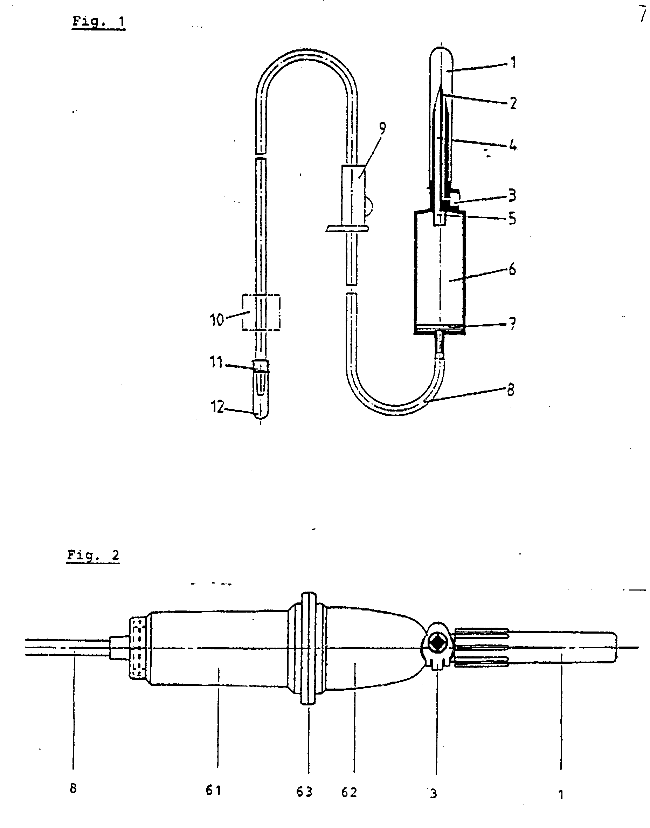

Die vorgenannte Aufgabe wird gelöst durch strahlensterilisierbare recyclierbare Infusions- und Transfusionsgeräte bestehend aus einer Schutzkappe (1), einem Einstechteil (2), einem Belüftungsteil mit Luftfilter und Ventilkörper (3), einem Flüssigkeitskanal (4), einem Tropfrohr (5), einer Tropfkammer (6), einem Flüssigkeitsfilter (7), einem Schlauch (8), einem Durchflußregler (9), einem Zuspritzteil (10), einem Anschlußstück mit Außenkegel (11) und einer Schutzkappe (12), die dadurch gekennzeichnet sind, daß sämtliche Bestandteile hergestellt sind aus thermoplastischen oder elastomeren Homopolymeren, Copolymeren, Blockcopolymeren auf der Basis von Styrolpolymerisat-freien Polyolefinen. Die Figur 1, die an die DIN 58362 angelehnt ist, gibt den prinzipiellen Aufbau eines Transfusionsgerätes wieder.

Mit Hilfe der vorliegenden Erfindung werden Infusions- und Transfusionsgeräte zur Verfügung gestellt, die chemisch inert, ethylenoxid- und gammasterilisierbar sind. Darüberhinaus sind die Geräte komplett ohne Zerlegung in die Einzelteile recyclierbar und ohne Schadstoffentwicklung durch Verbrennung entsorgbar. In die Ökobilanz gehen praktisch nur polyolefinische Werkstoffe ein, was sich durchweg in einer positiven Bewertung gegenüber anderen nicht polyolefinischen Werkstoffgruppen niederschlägt.

Polyolefine sind bekanntermaßen teilkristalline Thermoplaste, die sich durch eine gute chemische Beständigkeit und gute elektrische Isoliereigenschaften auszeichnen.

Hauptsächlich eingesetzt werden Polyethylene mit unterschiedlicher Dichte und Polypropylen. Für besondere Anforderungen werden spezielle Polyolefine verwendet. Spezialsorten umfassen beispielsweise pulverförmiges Polyethylen, LLD-Polyethylen, hochmolekulares Polyethylen, vernetztes Polyethylen PE-V. Spezialsorten der Polypropylene sind beispielsweise PP-Elastomer-Blends oder Polypropylen mit enger Molekülgrößenverteilung.

Unter speziellen Polyolefinen im Sinne der vorliegenden Erfindung sind beispielsweise Polybuten-1 und Poly(-4-methylpenten-1) zu verstehen. Weiterhin umfaßt der Begriff im Sinne der vorliegenden Erfindung Ionomere, d.h. Copolymerisate von Ethylen mit Acrylsäure, bei denen auch Ionenbindungen wirksam sind. Dadurch erhält man Kunststoffe mit sehr günstigen Eigenschaften, die je nach Shore Härte vor allem dort eingesetzt werden können, wo bisher Hart- und Weich-PVC oder Styrolpolymerisate eingesetzt worden sind.

In den derzeit im Handel erhältlichen Infusions- und Transfusionsgeräten werden überwiegend Schläuche aus Weich-PVC eingesetzt, die zum einen nicht patientengerecht sind, da sie Weichmacher enthalten, die an das Übertragungsgut und damit an den Patienten abgegeben werden können. Zum anderen sind derartige Schläuche nicht umweltgerecht, da sie bei der Entsorgung durch Verbrennung neben Kohlendioxid und Wasser umweltbelastenden Chlorwasserstoff erzeugen.

Mit Hilfe der vorliegenden Erfindung wurde nun gefunden, daß Verbindungsschläuche (8) aus Polyethylen mit sehr niedriger Dichte (PE-LLD) oder aus linearem Polyethylen ebenfalls mit sehr niedriger Dichte (PE-LVLD) hergestellt werden können. Eine Alternative zu Polyethylen mit sehr niedriger Dichte ist beispielsweise ein Ethylen/Vinylacetat-Copolymer (EVA), ebenso wie spezielle Ionomere. Mit Hilfe der vorliegenden Erfindung konnte auch das Problem der schwierigen Verbindungstechnik beim Fügen von Polyolefinschläuchen gelöst werden. Diese Polyolefine sind in der Regel nur nach einer Korona- oder Plasmabehandlung miteinander zu verkleben. In Druckfestigkeitstests zeigte sich eine außerordentliche Stabilität der Fügestelle, wenn die Polyolefinschläuche unter Verwendung von organischen Lösungsmitteln, beispielsweise Cyclohexan mit den Schlauchansätzen gefügt werden. Alternativen zur Fügung der Polyolefinschläuche bestehen beispielsweise in einer Ultraschallverschweißung oder durch Verklebung mittels Licht- oder UV-härtbaren Klebstoffen. Voraussetzung ist jedoch, daß die Fügestelle den Anforderungen gerecht wird, einem Innendruck von 6 bar wenigstens 15 min zu überstehen. Weitere Anforderungen an entsprechende Schläuche, die mit Hilfe der vorliegenden Erfindung erfüllt werden können, sind beispielsweise ein positives Knickverhalten, d. h. der Schlauch darf bei dem angewandten Wicklungsverfahren nicht ausknicken. Der Schlauch wird durch den total abgeklemmten Zustand (24 Stunden) nicht beschädigt oder bleibend verformt. Der Schlauch kann mit der Rollenklemme so bedient werden, daß unterschiedliche Tropfenzahlen einstellbar sind, wobei die Totalabklemmung möglich ist. Der Rollwiderstand ist bei Betätigung der Rollenklemme nicht größer als bei gegebenem PVC-Werkstoff. Das Schlauchmaterial ist so durchsichtig, daß eventuelle Lufteinschlüsse im Volumenstrom einwandfrei erkannt werden können. Die Rückerinnerung des Materials entspricht handelsüblichem PVC-Material. Der Schlauch kann mit bestehenden Technologien (Montagemaschinen und Schneidemaschinen) und gleichen Vorgabezeiten verarbeitet werden. Die chemischen und biologischen Anforderungen nach DIN 58362 werden ebenso erfüllt, wie der Hämolysetest und die Pyrogenfreiheit gemäß dieser DIN-Vorschrift. Der Schlauch ist sowohl durch Gammastrahlung als auch durch Ethylenoxid sterilisierbar, wobei die Eigenschaften im wesentlichen erhalten bleiben.

In einer weiteren bevorzugten Ausführungsform der vorliegenden Erfindung wird der Ventilkörper des Belüftungsteils (3) und das elastische Zuspritzteil (10) jeweils unabhängig voneinander aus einem Ethylen/Propylen-Dien (EPDM) hergestellt. Bisher wurden die Luftfilter des Belüftungsteiles (3) im wesentlichen aus Borosilikatglasfaser hergestellt. Im Rahmen der vorliegenden Erfindung wird jedoch bevorzugt das Belüftungsfilter aus einem Polyethylenvlies oder einem entsprechenden Gewebe hergestellt.

Im Sinne der vorliegenden Erfindung ist es besonders bevorzugt, daß das Luftfilter des Belüftungsteils (3) aus einem Polyethylen-Vlies, einem entsprechenden Gewebe oder einer mikroporösen Filterscheibe hergestellt ist.

Weiterhin ist es im Sinne der vorliegenden Erfindung bevorzugt, Infusions- und Transfusionsgeräte zur Verfügung zu stellen, wobei die Schutzkappe (1, 12), das Tropfkammerunterteil (61), das Belüftungsteil (3) das Gehäuse des Durchflußreglers (9), einschließlich des Durchflußreglerrades und das Anschlußstück mit Außenkegel (11) jeweils aus Polyethylen verschiedener Dichte oder auch Poly(-4-methylpenten-1) hergestellt sind.

Besonders bevorzugt im Sinne der vorliegenden Erfindung ist es, wenn das Gehäuse des Durchflußreglers (9) aus Poly-(-4-methylpenten-1) oder Polyethylen mit hoher Dichte (PE-HD) und das Durchflußreglerrad aus Polyethylen mit hoher Dichte (PE-HD) hergestellt sind.

Die Figur 2 gibt eine bevorzugte Ausführungsform der vorliegenden Erfindung wieder, bei der die Tropfkammer unterteilt ist in ein Tropfkammerunterteil (61), ein Tropfkammeroberteil (62) und einen Umspritzring (63).

In einer weiteren bevorzugten Ausführungsform der vorliegenden Erfindung sind die Infusions- und Transfusionsgeräte dadurch gekennzeichnet, daß das Tropfkammeroberteil (62), das Gehäuse des Durchflußreglers (9) und das Anschlußstück mit Außenkegel (11) aus Poly(-4-methylpenten-1) und der Umspritzungsring (63) aus einem Polyethylen mit hoher Dichte hergestellt sind.

Ein besonderes Problem stellt auch das Flüssigkeitsfilter (7) dar. Das Flüssigkeitsfilter (7) muß bei der Prüfung der Wirksamkeit mit einer wäßrigen Suspension handelsübliche Latexpartikel mit einem Durchmesser von 20 µm wenigstens 80 % der Latexpartikel zurückhalten. In einer besonderen Ausführungsform der vorliegenden Erfindung ist daher die Filterscheibe hergestellt aus einem Faservlies oder -gewebe mit einem Filamentdurchmesser von etwa 15 µm. Besonders bevorzugte Monofile sind beispielsweise ausgewählt aus Polyethylen und/oder Polypropylen.

Das Zuspritzteil (10) ermöglicht eine Injektion in das Schlauchlumen. Daher wird das Zuspritzteil (10) vorzugsweise in der Nähe des Anschlußstückes mit Außenkegel (11) angebracht. Besonders bevorzugte Materialien für das Zuspritzteil sind beispielsweise Blockcopolymere aus Polyethylen/Butylen oder auch andere thermoplastische Elastomere auf Styrolpolymerisat-freier polyolefinischer Basis.

Besonders bevorzugtes Material für das Anschlußstück mit Außenkegel (11) sind die oben genannten Ionomere. Ein besonders bevorzugtes Material für das Einstechteil (2) ist beispielsweise Poly(-4-methylpenten-1).

Durch den Einsatz von Polyolefinen, die im Vergleich zu anderen Thermoplasten ein sehr niedriges spezifisches Gewicht aufweisen, werden darüberhinaus im logistischen Bereich Vorteile geschaffen. Im Vergleich zum herkömmlichen Gerät, das aus mehreren Polymeren besteht, wird eine Gewichtseinsparung von bis zu 25 % erzielt.

Da beispielsweise nach dem Deutschen Arzneimittelbuch (DAB9) keine Kunststoffabfälle zur Herstellung von Behältnissen für pharmazeutische Produkte oder auch für die Produkte selbst, die mit Infusionslösungen und Blut in Kontakt kommen, eingesetzt werden dürfen, sind die Recyclate beispielsweise für die Herstellung von Aufhängevorrichtungen für Glasflaschen, Halterungen von Dialysemodulen, Boxen für gebrauchte Kanülen sowie Kanister im nichtmedizinischen Bereich einsetzbar.

## Patentansprüche

1. Strahlensterilisierbare recyclierbare Infusions- und Transfusionsgeräte bestehend aus einer Schutzkappe (1), einem Einstechteil (2), einem Belüftungsteil mit Luftfilter und Ventilkörper (3), einem Flüssigkeitskanal (4), einem Tropfrohr (5), einer Tropfkammer (6), einem Flüssigkeitsfilter (7), einem Schlauch (8), einem Durchflußregler (9), einem Zuspritzteil (10), einem Anschlußstück mit Außenkegel (11) und einer Schutzkappe (12), die dadurch gekennzeichnet sind, daß sämtliche Bestandteile hergestellt sind aus thermoplastischen oder elastomeren Homopolymeren, Copolymeren, Blockcopolymeren auf der Basis von Styrolpolymerisat-freien Polyolefinen.

2. Infusions- und Transfusionsgeräte nach Anspruch 1, dadurch gekennzeichnet, daß der Schlauch (8) aus einem Polyethylen mit sehr niedriger Dichte (PE-VLD), oder aus einem linearen Polyethylen mit sehr niedriger Dichte (PE-LVLD) oder einem Ethylen/Vinylacetat-Copolymer (EVA) hergestellt ist.

3. Infusions- und Transfusionsgeräte nach Anspruch 1 oder 2, dadurch gekennzeichnet, daß der Ventilkörper des Belüftungsteiles (3) und das Zuspritzteil (10) aus einem Ethylen/Propylen-Dien (EPDM) hergestellt ist.

4. Infusion- und Transfusionsgeräte nach einem oder mehreren der Ansprüche 1 bis 3, dadurch gekennzeichnet, daß das Luftfilter des Belüftungsteils (3) aus einem Polyethylen-Vlies, einem entsprechenden Gewebe oder einer mikroporösen Filterscheibe hergestellt ist.

5. Infusions- und Transfusionsgeräte nach einem oder mehreren der Ansprüche 1 bis 4, dadurch gekennzeichnet, daß die Schutzkappe (1, 12), das Tropfkammerunterteil (61), das Belüftungsteil (3), der Durchflußregler (9), einschließlich Durchflußreglerrad und das Anschlußstück mit Außenkegel (11) jeweils aus Polyethylen unterschiedlicher Dichte hergestellt sind.

6. Infusions- und Transfusionsgeräte nach einem oder mehreren der Ansprüche 1 bis 5, dadurch gekennzeichnet, daß die Tropfkammer (6) aus einem Tropfkammeroberteil (62), einem Tropfkammerunterteil (61) und einem Umspritzungsring (63) besteht.

7. Infusions- und Transfusionsgeräte nach Anspruch 6, dadurch gekennzeichnet, daß das Tropfkammeroberteil und das Anschlußstück mit Außenkegel (11) aus einem Poly(-4-methylpenten-1) hergestellt sind.

8. Infusions- und Transfusionsgeräte nach einem oder mehreren der Ansprüche 1 bis 7, dadurch gekennzeichnet, daß das Gehäuse des Durchflußreglers (9) aus Poly(-4-methylpenten-1) oder Polyethylen mit hoher Dichte (PE-HD) und das Durchflußreglerrad aus Polyethylen mit hoher Dichte (PE-HD) hergestellt sind.

## Claims

1. Radiation sterilizable recyclable infusion and transfusion devices consisting of a protecting cap (1), a piercing member (2), an aeration member with air filter and valve body (3), a liquid channel (4), a dropper tube (5), a dropping chamber (6), a liquid filter (7), a flexible tube (8), a flow control valve (9), an injection member (10), a connecting piece with a male taper (11) and a protecting cap (12), characterized in that all components are prepared from thermoplastic or elastomeric homopolymers, copolymers, block copolymers based on polyolefins which are free from styrene polymerizate.

2. The infusion and transfusion devices according to claim 1, characterized in that said flexible tube (8) is prepared from very low density polyethylene (PE-VLD), or from very low density linear polyethylene (PE-LVLD), or from an ethylene/vinyl acetate copolymer (EVA).

3. The infusion and transfusion devices according to claim 1 or 2, characterized in that said valve body of said aeration member (3) and said injection member (10) are prepared from an ethylene/propylene-diene (EPDM).

4. The infusion and transfusion devices according to one or more of claims 1 to 3, characterized in that said air filter of said aeration member (3) is prepared from a polyethylene web, a corresponding fabric or a microporous filter disk.

5. The infusion and transfusion devices according to one or more of claims 1 to 4, characterized in that said protecting caps (1, 12), the bottom part of said dropping chamber (61), said aeration member (3), said flow control valve (9) including a flow controller wheel, and said connecting piece with a male taper (11) are respectively prepared from polyethylenes of different densities.

6. The infusion and transfusion devices according to one or more of claims 1 to 5, characterized in that said dropping chamber (6) consists of a dropping chamber top part (62), a dropping chamber bottom part (61), and a molded-on ring (63).

7. The infusion and transfusion devices according to claim 6, characterized in that said dropping chamber top part and said connecting piece with a male taper (11) are prepared from poly(4-methyl-pentene-1).

8. The infusion and transfusion devices according to one or more of claims 1 to 7, characterized in that the housing of said flow control valve (9) is prepared from poly(4-methyl-pentene-1) or high density polyethylene (PE-HD), and said flow controller wheel is prepared from high density polyethylene (PE-HD).

## Revendications

1. Appareil de perfusion et de transfusion stérilisable par irradiation et recyclable, se composant d'un capuchon protecteur (1), d'un élément pour piquer (2), d'un élément d'aération avec un filtre à air et un corps de soupape (3), d'a canal pour liquides (4), d'un tube d'égouttage (5), d'une chambre d'égouttage (6), d'un filtre à liquides (7), d'un tuyau flexible (8), d'un régulateur d'écoulement (9), d'un élément d'injection (10), d'une pièce de raccordement à cône extérieur (11) et d'un capuchon protecteur (12), caractérisé en ce que l'ensemble des composants sont fabriqués à partir d'homopolymères, de copolymères, de copolymères séquencés, thermoplastiques ou élastomères, à base de polyoléfines sans produit de polymérisation du styrène.

2. Appareil de perfusion et de transfusion selon la revendication 1, caractérisé en ce que le tuyau flexible (8) est fabriqué à partir d'un polyéthylène très basse densité (PE-VLD) ou d'un polyéthylène linéaire très basse densité (PE-LVLD) ou d'un copolymère éthylène/acétate de vinyle (EVA).

3. Appareil de perfusion et de transfusion selon la revendication 1 ou 2, caractérisé en ce que le corps de soupape de l'élément d'aération (3) et l'élément d'injection (10) sont fabriqués à partir d'un diène éthylène/propylène (EPDM).

4. Appareil de perfusion et de transfusion selon l'une ou plusieurs des revendications 1 à 3, caractérisé en ce que le filtre à air de l'élément d'aération (3) est fabriqué à partir d'un non-tissé en polyéthylène, d'un tissu correspondant ou d'un disque de filtre microporeux.

5. Appareil de perfusion et de transfusion selon l'une ou plusieurs des revendications 1 à 4, caractérisé en ce que le capuchon protecteur (1, 12), la partie inférieure (61) de la chambre d'égouttage, l'élément d'aération (3), le régulateur d'écoulement (9), y compris la molette du régulateur d'écoulement, et la pièce de raccordement a cône extérieur (11) sont respectivement fabriqués à partir de polyéthylènes de densités différentes.

6. Appareil de perfusion et de transfusion selon l'une ou plusieurs des revendications 1 à 5, caractérisé en ce que la chambre d'égouttage (6) se compose d'une partie supérieure de chambre d'égouttage (62), d'une partie inférieure de chambre d'égouttage (61) et d'une bague d'injection (63).

7. Appareil de perfusion et de transfusion selon la revendication 6, caractérisé en ce que la partie supérieure de la chambre d'égouttage et la pièce de raccordement à cône extérieur (11) sont fabriquées à partir d'un poly(-4-méthylpentène-1).

8. Appareil de perfusion et de translusion selon l'une ou plusieurs des revendications 1 à 7, caractérisé en ce que le boîtier du régulateur d'écoulement (9) est fabriqué à partir d'un poly(-4-méthylpentène-1) ou d'un polyéthylène haute densité (PE-HD) et la molette du régulateur d'écoulement à partir d'un polyéthylène haute densité (PE-HD).
